# EUROPEAN PATENT APPLICATION

(11) **EP 4 678 650 A1**
(43) Date of publication of application: **14.01.2026**
(21) Application number: 24788822.5
(22) Date of filing: 11.04.2024
(51) Int. Cl.: C07K 1/22, C07K 19/00, C12P 21/02, C12Q 1/02

(54) **METHOD FOR PREPARING POLYPEPTIDE SOLUTION FOR EVALUATING PHYSIOLOGICAL ACTIVITY, AND METHOD FOR EVALUATING PHYSIOLOGICAL ACTIVITY OF POLYPEPTIDE**

(30) Priority: 11.04.2023 JP 2023064440
(71) Applicant: FUJIFILM Corporation, Tokyo 106-8620 (JP)
(72) Inventor: IZUTA, Shin, Ashigarakami-gun, Kanagawa 258-8577 (JP); MASUI, Misaki, Ashigarakami-gun, Kanagawa 258-8577 (JP); HABA, Ryota, Ashigarakami-gun, Kanagawa 258-8577 (JP)
(74) Representative: Hoffmann Eitle
(86) International application number: PCT/JP2024/014746
(87) International publication number: WO 2024/214800

(57) **Abstract**

A method for preparing a polypeptide solution for evaluating a physiological activity and A method for evaluating a physiological activity of a polypeptide, the method includes: (A) bringing a crude polypeptide solution after cell-free expression into contact with a carrier to bind a polypeptide having a pH-responsive affinity tag to the carrier; (B) eluting the polypeptide bound to the carrier with an acidic aqueous solution or a basic aqueous solution, each having a concentration of less than 50 mM, to obtain an eluted solution; and (C) adding a basic aqueous solution or an acidic aqueous solution to the eluted solution to neutralize an acid or a base contained in the eluted solution, and neutralizing in such a manner that a concentration of a salt formed by a neutralization reaction is less than 50 mM, to obtain a polypeptide solution.

## Description

### BACKGROUND OF THE INVENTION

### 1. Field of the Invention

The present disclosure relates to a method for preparing a polypeptide solution for evaluating a physiological activity and a method for evaluating a physiological activity of a polypeptide.

### 2. Description of the Related Art

Medium-molecular-weight compounds, including polypeptides, have attracted attention as compounds that possess low manufacturing cost and high stability, which are features of low-molecular-weight compounds, as well as a binding property to target proteins, which is a feature of high-molecular-weight compounds such as antibodies, and have begun to be used in a wide range of bio-related industries, including therapeutic and diagnostic drugs. Among these, special polypeptides that can achieve high binding properties to target proteins and stability by incorporating an unnatural amino acid that does not naturally exist have been actively developed.

In recent years, special polypeptides that not only bind to proteins but also exhibit a physiological activity such as cell proliferation via binding to proteins have begun to attract attention as novel highly functional compounds. However, chemical synthesis of special polypeptides requires a cost and a period of time, and special polypeptides exhibiting a physiological activity is only a small part of special polypeptides having protein binding properties. Therefore, it has been extremely difficult to explore the special polypeptides.

As a method for exploring polypeptides that exhibit a physiological activity on cells, WO2022/158554A reports a method for rapidly and conveniently exploring a physiological activity on cells by applying a polypeptide synthesized using a cell-free expression method on cells after dilution, which is a rapid and low-cost method.

A method of purifying a polypeptide synthesized using a cell-free expression method and using the polypeptide for an intended application is also known. In Method for purifying His-tagged protein, [online], 2021, Gene Frontier Corporation, [Searched on March 20, 2023], Internet <URL: https://www.genefrontier.com/cases/purefrex/applications-09/)>, affinity purification of a polypeptide expressed using a cell-free expression is described.

### SUMMARY OF THE INVENTION

In a case where a special polypeptide incorporating an unnatural amino acid is synthesized by a cell-free expression method, the concentration of the synthesized polypeptide is extremely low. Therefore, as described in WO2022/158554A, in a case where dilution is performed after cell-free expression, it is difficult to secure a sufficient concentration for activity evaluation. On the other hand, in a case where a polypeptide solution after the cell-free expression is subjected to the evaluation of the physiological activity on cells without performing the dilution, due to the cytotoxicity of the components contained in the polypeptide solution after the cell-free expression, the cells die or are damaged, making evaluation of physiological activity difficult. Therefore, the method of WO2022/158554A is difficult to apply to the special polypeptide in which the unnatural amino acid is incorporated.

To remove toxic components from the polypeptide solution after the cell-free expression, it may also be considered to perform affinity purification, as described in Method for purifying His-tagged protein, [online], 2021, Gene Frontier Corporation, [Searched on March 20, 2023], Internet <URL: https://www.genefrontier.com/cases/purefrex/applications-09/)>. However, in affinity purification that is generally used, the eluent has cytotoxicity due to contamination of a high concentration of imidazole, contamination of a high concentration with polypeptides for elution, a low pH, and the like. Therefore, the eluted solution cannot be used as it is for the evaluation of physiological activity on cells. In a case where a step of removing contaminants is separately performed, the loss of the polypeptide is accompanied, and the additional cost is also required.

In view of such a situation, the present disclosure relates to a method of preparing a polypeptide solution for evaluating physiological activity and a method of evaluating the physiological activity of a polypeptide, both of which enable convenient evaluation of the physiological activity of a polypeptide synthesized by cell-free expression, at a high polypeptide concentration.

The means for achieving the above-mentioned object includes the following aspects.
<1> A method for preparing a polypeptide solution for evaluating physiological activity, the method comprising:
   (A) bringing a crude polypeptide solution after cell-free expression into contact with a carrier to bind a polypeptide having a pH-responsive affinity tag to the carrier;
   (B) eluting the polypeptide bound to the carrier with an acidic aqueous solution or a basic aqueous solution, each having a concentration of less than 50 mM, to obtain an eluted solution; and
   (C) adding a basic aqueous solution or an acidic aqueous solution to the eluted solution to neutralize an acid or a base contained in the eluted solution, and neutralizing in such a manner that a concentration of a salt formed by a neutralization reaction is less than 50 mM, to obtain a polypeptide solution.
<2> A method for evaluating physiological activity of a polypeptide, the method comprising:
   preparing a polypeptide solution by the method according to <1>; and
   bringing the polypeptide solution into contact with a cell to evaluate the physiological activity of the polypeptide.
<3> The method according to <1> or <2>, in which the polypeptide contains an unnatural amino acid residue.
<4> The method according to any one of <1> to <3>, in which the polypeptide contains one or more ring structures.
<5> The method according to <4>, in which the ring structure includes the following structure, 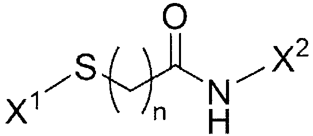 in the formula, X¹ and X² each independently represent any linking group, and n represents an integer of 1 to 10.
<6> The method according to <4> or <5>, in which the polypeptide contains 1 to 4 ring structures.
<7> The method according to any one of <1> to <6>, in which, in the step (B), an acidic aqueous solution having a concentration of less than 50 mM is used, and, in the step (C), a basic aqueous solution is used.
<8> The method according to <7>, in which the acidic aqueous solution is an aqueous solution of hydrochloric acid.
<9> The method according to <7> or <8>, in which the basic aqueous solution is an aqueous solution of sodium bicarbonate, an aqueous solution of sodium carbonate, or a mixed aqueous solution of sodium bicarbonate and sodium carbonate.
<10> The method according to any one of <1> to <9>, in which the carrier is magnetic beads.
<11> The method according to any one of <1> to <10>, in which the affinity tag is a FLAG tag, an HA tag, a His tag, a c-Myc tag, a V5 tag, a Strep tag, or a PA tag.
<12> The method according to any one of <1> to <11>, further comprising:
   expressing the polypeptide by cell-free expression before (A).
<13> The method according to any one of <1> to <12>, further comprising:
   washing the carrier with water after (A) and before (B).

According to the present disclosure, there are provided a method of preparing a polypeptide solution for evaluating physiological activity and a method of evaluating the physiological activity of a polypeptide, both of which enable convenient evaluation of the physiological activity of a polypeptide synthesized by cell-free expression, at a high polypeptide concentration.

### DESCRIPTION OF THE PREFERRED EMBODIMENTS

Hereinafter, embodiments according to the present disclosure will be described. These descriptions and Examples are only illustrative of the embodiments and do not limit the ranges of the embodiments. The action mechanism mentioned in the present disclosure includes estimation, and the accuracy thereof does not limit the ranges of the embodiments.

The term "step" or a term representing a step in the present disclosure includes not only a step independent of another step, but also a step that achieves a desired effect of the step even in a case of not being clearly distinguished from the other step.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. In addition, in the numerical ranges described in the present disclosure, the upper limit value or the lower limit value of the numerical range may be replaced with the value shown in Examples.

In the present disclosure, each component may include a plurality of types of substances corresponding thereto. In the present disclosure, upon referring to an amount of each component in a composition, the amount means a total amount of a plurality of types of substances present in the composition unless otherwise specified, in a case where a plurality of types of substances corresponding to each component are present in the composition.

In the present disclosure, in the notation of an amino acid, the three-letter notation and the one-letter notation established by IUPAC-IUBMB joint commission on Biochemical Nomenclature (IUPAC-IUBMB JCBN) are used. Unless otherwise specified, the amino acid referred to in the present disclosure is an L-amino acid.

In the present disclosure, the unit "M" is a unit of molar concentration and is synonymous with mol/L.

In the present disclosure, unless otherwise specified, even in a case where an element is expressed in a singular form, a plurality of elements are not excluded unless a technical contradiction occurs.

### <Method for preparing polypeptide solution for evaluating physiological activity and method for evaluating physiological activity of polypeptide>

In one embodiment of the present disclosure, a method of preparing a polypeptide solution for evaluating physiological activity includes (A) bringing a crude polypeptide solution after cell-free expression into contact with a carrier to bind a polypeptide having a pH-responsive affinity tag to the carrier, (B) eluting the polypeptide bound to the carrier with an acidic aqueous solution or a basic aqueous solution, each having a concentration of less than 50 mM, to obtain an eluted solution, and (C) adding a basic aqueous solution or an acidic aqueous solution to the eluted solution to neutralize an acid or a base contained in the eluted solution, and neutralizing in such a manner that a concentration of a salt formed by a neutralization reaction is less than 50 mM, to obtain a polypeptide solution.

In one embodiment of the present disclosure, the method of evaluating the physiological activity of a polypeptide includes preparing a polypeptide solution by the method of preparing a polypeptide solution for evaluating a physiological activity described above, and (D) bringing the polypeptide solution into contact with a cell to evaluate the physiological activity of the polypeptide.

In the method of preparing a polypeptide solution and the method of evaluating a physiological activity according to the present disclosure (hereinafter, both are also collectively referred to as a "method of the present disclosure"), a polypeptide after cell-free expression is purified using affinity purification. Affinity purification refers to a purification method in which a molecule to be purified is specifically bound to a carrier by a non-covalent bond, and then eluted from the carrier. According to the method of the present disclosure, it is possible to conveniently evaluate the physiological activity of the polypeptide at a high polypeptide concentration. In the method of the present disclosure, the polypeptide bound to the carrier is eluted with an acidic aqueous solution or a basic aqueous solution, each having a concentration of less than 50 mM, and then the eluted solution is neutralized in such a manner that the salt concentration is less than 50 mM. The obtained polypeptide solution can be evaluated for the physiological activity under conditions in which the influence of the osmotic pressure is small due to the sufficiently low salt concentration, and hardly contains components other than the salt. In addition, since the cytotoxic components contained in the polypeptide solution after the cell-free expression are sufficiently reduced, even in a case where the evaluation of the physiological activity on cells is performed as it is, the evaluation can be performed while reducing the influence of cytotoxicity. In this manner, it is also possible to rapidly prepare a polypeptide solution at a low cost and evaluate the physiological activity.

Hereinafter, the above-described steps of (A) to (D) will also be referred to as steps (A) to (D), respectively. In addition, the step (A) may also be referred to as an "binding step", the step (B) may also be referred to as an "elution step", the step (C) may also be referred to as a "neutralization step", and the step (D) may also be referred to as an "evaluation step".

The method of the present disclosure may further include other steps in addition to the steps (A) to (D). For example, the method of the present disclosure may further include a step of washing the carrier with water after the step (A) and before the step (B). In addition, the method of the present disclosure may further include expressing the polypeptide by cell-free expression before the step (A).

Hereinafter, each step in the method of the present disclosure will be described.

### [Cell-free expression]

The method of the present disclosure may include expressing a polypeptide by cell-free expression before (A). The term "Cell-free expression" refers to a method of performing in vitro expression of a polypeptide from template nucleic acids by using components (ribosome, transcription/translation factor, and the like) derived from viable cells or obtained by a genetic engineering method, instead of using cells (such as Escherichia coli).

The cell-free expression has the following advantages as compared with the expression using cells such as Escherichia coli. First, since it is not necessary to maintain viable cells, the operability is good and the degree of freedom of the system is high. Therefore, it is possible to design a synthetic system in which various modifications are performed depending on the properties of the target polypeptide. Secondly, a polypeptide that is toxic to the cells to be used generally cannot be synthesized in an expression system using cells, but in a cell-free expression system, a polypeptide that has cytotoxicity can also be synthesized. Thirdly, since it is possible to simultaneously and rapidly synthesize various polypeptides and to easily separate and purify the produced polypeptides, high throughput is easily achieved. Fourthly, it is possible to synthesize a special polypeptide containing an unnatural amino acid.

### -Polypeptide-

The polypeptide to be expressed by cell-free expression is a polypeptide in which a pH-responsive affinity tag is linked to a polypeptide that is a subject of physiological activity evaluation (hereinafter, also referred to as a "polypeptide to be evaluated"). In the present disclosure, a polypeptide refers to a molecule in which amino acids are linked by a peptide bond. The number of amino acid residues of the polypeptide is not limited, and the polypeptide is a term including a protein.

The polypeptide to be evaluated is any polypeptide for which the physiological activity on cells is desired to be evaluated. In the present disclosure, the term "physiological activity" of a polypeptide refers to a physiological activity on viable cells. Examples of the physiological activity include activities on cell proliferation, cell differentiation status, cell viability, signals of various intracellular proteins, responses of various reporters, and the like. In the evaluation step, the polypeptide to be evaluated is brought into contact with the cell, and a change in the state of the cell is observed, whereby the evaluation of the physiological activity of the polypeptide to be evaluated can be performed.

From the viewpoint of the polypeptide activity expression, the number of amino acid residues of the polypeptide to be evaluated is preferably 6 or more. From the viewpoint of expression efficiency, the number of amino acid residues of the polypeptide to be evaluated is preferably less than 600, more preferably less than 300, and still more preferably less than 150. From such viewpoints, the number of amino acid residues of the polypeptide to be evaluated is preferably 6 or more and less than 600, more preferably 6 or more and less than 300, and still more preferably 6 or more and less than 150.

The pH-responsive affinity tag refers to a tag that causes the molecule to be purified to exhibit a property of binding to a carrier at a specific pH and dissociating from the carrier at another specific pH. From the viewpoint of the stability of the polypeptide, the affinity tag is preferably an affinity tag that bind to a carrier at a neutral pH (for example, a pH of 6 to 8) and dissociates from the carrier at an acidic pH (for example, a pH of 4 or less).

Any affinity tag can be used as long as it exhibits pH responsiveness, but from the viewpoint of minimizing the effect on the physiological activity of the polypeptide to be evaluated, a tag composed of a small number of amino acids is preferable. Examples of the tag having a small number of amino acids include a FLAG tag, an HA tag, a His tag, a c-Myc tag, a V5 tag, a Strep tag, and a PA tag. Among these, from the viewpoint of purification yield, the HA tag, the His tag, the c-Myc tag, or the PA tag is more preferable.

In a case of expressing the polypeptide by cell-free expression, a spacer sequence may or may not be included at the N-terminus, the C-terminus, and/or in the sequence of the polypeptide sequence. From the viewpoint of purification yield, it is preferable to insert a spacer sequence before and after the affinity tag. In addition, from the viewpoint of minimizing the effect on the physiological activity of the polypeptide to be evaluated, it is preferable to insert a spacer sequence between the polypeptide to be evaluated and the affinity tag. Examples of the spacer sequence include a GGS linker.

In one aspect, it is preferable that the polypeptide to be expressed includes a translation-promoting sequence in the N-terminal translation region. By including the translation-promoting sequence in the polypeptide, the cell-free expression level of the nucleotide sequence encoding the polypeptide is improved. As the translation-promoting sequence, a nucleotide sequence encoding the following amino acid sequence (1) is preferable. Hereinafter, the polypeptide having the amino acid sequence (1) is also referred to as a "VKKX tag".

### Amino acid sequence (1): Val-Lys-Lys-(Xaa)ₙ

In the amino acid sequence (1), (Xaa)ₙ is a chain of n pieces of any amino acids, where n is an integer of 1 to 8, and the amino acids composing (Xaa)ₙ may be one type or two or more types.

As the amino acid composing (Xaa)ₙ, n pieces of amino acids selected from the group consisting of Ile, Lys, Arg, His, Ser, Thr, Asp, Cys, Asn, Tyr, Gln, Trp, and Phe are preferable, and n pieces of amino acids selected from the group consisting of Ile, Lys, Arg, His, Ser, Thr, and Asp are more preferable.

In the amino acid sequence (1), n is preferably an integer of 1 to 7 and more preferably an integer of 2 to 7.

As the VKKX tag, a polypeptide having an amino acid sequence of the amino acid sequence (2) is preferable, a polypeptide having an amino acid sequence of the amino acid sequence (3) is more preferable, and a polypeptide having an amino acid sequence of the amino acid sequence (4) is still more preferable.

### Amino acid sequence (2): Val-Lys-Lys-Xaa-(Xaa)ₘ

In the amino acid sequence (2), Xaa at the fourth position from an N-terminus is one amino acid selected from the group consisting of Ile, Lys, Arg, His, Ser, and Thr, (Xaa)ₘ is a chain of m pieces of any amino acids, where m is an integer of 0 to 7, and the amino acids composing (Xaa)ₘ may be one type or two or more types.

In the amino acid sequence (2), Xaa at the fourth position from the N-terminus is preferably one amino acid selected from the group consisting of Ile, Lys, and Thr, and more preferably Thr.

In the amino acid sequence (2), as the amino acid composing (Xaa)ₘ, m pieces of amino acids selected from the group consisting of Ile, Lys, Arg, His, Ser, Thr, Asp, Cys, Asn, Tyr, Gln, Trp, and Phe are preferable, and m pieces of amino acids selected from the group consisting of Ile, Lys, Arg, His, Ser, Thr, and Asp are more preferable.

In the amino acid sequence (2), m is preferably an integer of 0 to 6 and more preferably an integer of 1 to 6.

### Amino acid sequence (3): Val-Lys-Lys-Xaa-(Xaa)ₖ

In the amino acid sequence (3), Xaa at the fourth position from the N-terminus is one amino acid selected from the group consisting of Ile, Lys, and Thr, (Xaa)ₗ is a chain of k pieces of amino acids selected from the group consisting of Lys, Thr, and Asp, where k is an integer of 0 to 6, and the amino acids constituting (Xaa)ₗ may be one type or two or more types.

In the amino acid sequence (3), Xaa at the fourth position from the N-terminus is preferably Thr.

In the amino acid sequence (3), k is preferably an integer of 1 to 6.

### Amino acid sequence (4): Val-Lys-Lys-Thr-Lys-Thr-(Xaa)ⱼ (SEQ ID NO: 40)

In the amino acid sequence (4), (Xaa)ⱼ is a chain of j pieces of amino acids selected from the group consisting of Lys, Thr, and Asp, where j is an integer of 0 to 4, and the amino acids composing (Xaa)ⱼ may be one type or two or more types.

The nucleotide sequence encoding the VKKX tag is not limited. The nucleotide sequence of the VKKX tag is designed according to a codon table of a biosynthesis system (for example, a cell-free expression system) that is used for the expression of the tagged polypeptide.

Examples of the VKKX tag and the nucleotide sequence encoding the VKKX tag are shown in the table below. The nucleotide sequence encoding each VKKX tag is not limited to the sequences in the table below.

**[Table 1]**

| VKKX tag | | Nucleotide sequence encoding VKKX tag (example) | |
|---|---|---|---|
| SEQ ID NO | Amino acid sequence | SEQ ID NO | Nucleotide sequence |
| 41 | VKKI | 87 | GTTAAAAAAATA |
| 42 | VKKK | 88 | GTTAAAAAAAAA |
| 43 | VKKT | 89 | GTTAAAAAAACA |
| 44 | VKKR | 90 | GTTAAAAAACGG |
| 45 | VKKS | 91 | GTTAAAAAATCA |
| 46 | VKKH | 92 | GTTAAAAAACAT |
| 47 | VKKM | 93 | GTTAAAAAAATG |
| 48 | VKKP | 94 | GTTAAAAAACCT |
| 49 | VKKG | 95 | GTTAAAAAAGGC |
| 50 | VKKL | 96 | GTTAAAAAACTA |
| 51 | VKKV | 97 | GTTAAAAAAGTT |
| 52 | VKKE | 98 | GTTAAAAAAGAA |
| 53 | VKKA | 99 | GTTAAAAAAGCA |
| 54 | VKKD | 100 | GTTAAAAAAGAT |
| 55 | VKKC | 101 | GTTAAAAAATGT |
| 56 | VKKN | 102 | GTTAAAAAAAAT |
| 57 | VKKY | 103 | GTTAAAAAATAT |
| 58 | VKKQ | 104 | GTTAAAAAACAA |
| 59 | VKKW | 105 | GTTAAAAAATGG |
| 60 | VKKF | 106 | GTTAAAAAATTT |
| 61 | VKKTKT | 107 | GTTAAAAAAACAAAAACA |
| 62 | VKKTKTT | 108 | GTTAAAAAAACAAAAACAACA |
| 63 | VKKTKTD | 109 | GTTAAAAAAACAAAAACAGAT |
| 64 | VKKTKTKK | 110 | GTTAAAAAAACAAAAACAAAAAAA |
| 65 | VKKTKTKT | 111 | GTTAAAAAAACAAAAACAAAAACA |
| 66 | VKKTKTKD | 112 | GTTAAAAAAACAAAAACAAAAGAT |
| 67 | VKKTKTTK | 113 | GTTAAAAAAACAAAAACAACAAAA |
| 68 | VKKTKTTT | 114 | GTTAAAAAAACAAAAACAACAACA |
| 69 | VKKTKTDD | 115 | GTTAAAAAAACAAAAACAGATGAT |
| 70 | VKKTKTKKT | 116 | GTTAAAAAAACAAAAACAAAAAAAACA |
| 71 | VKKTKTKKD | 117 | GTTAAAAAAACAAAAACAAAAAAAGAT |
| 72 | VKKTKTKTK | 118 | GTTAAAAAAACAAAAACAAAAACAAAA |
| 73 | VKKTKTKTT | 119 | GTTAAAAAAACAAAAACAAAAACAACA |
| 74 | VKKTKTKDK | 120 | GTTAAAAAAACAAAAACAAAAGATAAA |
| 75 | VKKTKTKDT | 121 | GTTAAAAAAACAAAAACAAAAGATACA |
| 76 | VKKTKTKDD | 122 | GTTAAAAAAACAAAAACAAAAGATGAT |
| 77 | VKKTKTTKK | 123 | GTTAAAAAAACAAAAACAACAAAAAAA |
| 78 | VKKTKTTKT | 124 | GTTAAAAAAACAAAAACAACAAAAACA |
| 79 | VKKTKTTKD | 125 | GTTAAAAAAACAAAAACAACAAAAGAT |
| 80 | VKKTKTKKTK | 126 | GTTAAAAAAACAAAAACAAAAAAAACAAAA |
| 81 | VKKTKTKKDK | 127 | GTTAAAAAAACAAAAACAAAAAAAGATAAA |
| 82 | VKKTKTKTKK | 128 | GTTAAAAAAACAAAAACAAAAACAAAAAAA |
| 83 | VKKTKTKTTK | 129 | GTTAAAAAAACAAAAACAAAAACAACAAAA |
| 84 | VKKTKTKDKK | 130 | GTTAAAAAAACAAAAACAAAAGATAAAAAA |
| 85 | VKKTKTTTKK | 131 | GTTAAAAAAACAAAAACAACAACAAAAAAA |
| 86 | VKKTKTKTDTT | 132 | GTTAAAAAAACAAAAACAAAAACAGATACAACA |

As the details of the translation-promoting sequence, the details described in WO2023/048290A can be applied.

The three-dimensional structure, amino acid sequence, number of amino acid residues, type of amino acid, nucleotide sequence encoding the polypeptide, and the like, of the polypeptide to be expressed are not limited. The polypeptide includes a polypeptide in which an amino acid is post-translationally modified. Examples of the post-translational modification of an amino acid include phosphorylation, methylation, acetylation, glycosylation, lipidation, and the like.

In the present disclosure, the amino acid includes a natural amino acid, an unnatural amino acid, a modified amino acid, and a derivative thereof.

In the present disclosure, the natural amino acid refers to an amino acid that composes a general protein, and examples thereof include alanine (Ala, A), arginine (Arg, R), asparagine (Asn, N), aspartic acid (Asp, D), and cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V). The natural amino acid may be a natural product or an artificial product.

In the present disclosure, the unnatural amino acid refers to an amino acid other than the twenty types of amino acids described above, and includes a natural product or an artificial product. Examples of the unnatural amino acid include an amino acid having a haloacetyl group (for example, chloroacetylated amino acids such as chloroacetylated lysine or chloroacetylated diaminobutyric acid), an N-methyl amino acid (for example, N-methylalanine or N-methylphenylalanine), and the like.

Examples of the modified amino acid include a labeled amino acid, which is an amino acid bonded to a labeling compound, and the like. The labeling compound is a substance that can be detected by a biochemical, chemical, immunochemical, or electromagnetic detection method. Examples of the labeling compound include a coloring agent compound, a fluorescent substance, a chemiluminescent substance, a bioluminescent substance, an enzyme substrate, a coenzyme, an antigenic substance, a substance that binds to a specific protein, a magnetic substance, and the like. Examples of the labeled amino acid include, as classified according to function, a fluorescent-labeled amino acid, a photoresponsive amino acid, a photoswitch amino acid, a fluorescent probe amino acid, and the like.

The amino acid in the labeled amino acid and the labeling compound may be directly bonded to each other or may be bonded to each other through a spacer. Examples of the spacer include polyolefins such as polyethylene and polypropylene; polyethers such as polyoxyethylene, polyethylene glycol, and polyvinyl alcohol; polystyrene, polyvinyl chloride, polyester, polyamide, polyimide, polyurethane, polycarbonate, and the like.

Examples of the derivative of the natural amino acid, the unnatural amino acid, or the modified amino acid include hydroxy acids, mercapto acids, and carboxylic acids.

In one aspect, the polypeptide may be a polypeptide containing an unnatural amino acid residue. Examples of the polypeptide containing an unnatural amino acid residue include the polypeptide containing unnatural amino acids exemplified above. According to the method of the present disclosure, even a polypeptide containing an unnatural amino acid can be conveniently evaluated for the physiological activity at a high polypeptide concentration.

The polypeptide may be a linear polypeptide or a polypeptide containing a ring structure (hereinafter, also referred to as a "cyclic polypeptide"). Since the polypeptide containing a ring structure generally exhibits the higher physiological activity than a linear polypeptide, a polypeptide in which the part of the polypeptide to be evaluated is cyclic is preferable among the polypeptides to be expressed.

The cyclic polypeptide is preferably a polypeptide containing a structure in which a first amino acid residue and a second amino acid residue, between which four or more amino acid residues are interposed, are covalently bonded via a main chain or a side chain. The number of amino acid residues interposed between the first amino acid residue and the second amino acid residue may be, for example, 4 to 20. The covalent bond can be formed by a reaction between the first functional group contained in the first amino acid residue and the second functional group contained in the second amino acid residue.

The first functional group and the second functional group may be the same type of functional group or different types of functional groups as long as the first functional group and the second functional group are covalently bonded to each other. Examples of a combination of the first functional group and the second functional group include a combination of a thiol group and a chloroacetyl group, a combination of a thiol group and a thiol group, and a combination of a carboxy group in a side chain and an amino group in a side chain.

Examples of the amino acid having a thiol group include cysteine.

Examples of the amino acid having a chloroacetyl group include chloroacetyl diaminobutyric acid and chloroacetylated lysine.

Examples of the amino acid having a carboxy group in the side chain include aspartic acid and glutamic acid.

Examples of the amino acid having an amino group in the side chain include lysine, asparagine, and glutamine.

The polypeptide may contain one or more ring structures. The number of ring structures contained in the polypeptide is preferably large from the viewpoint of expressing the physiological activity on cells, and is preferably small from the viewpoint of the yield of the cell-free expression. From such viewpoints, the number of ring structures contained in the polypeptide is preferably 1 to 4, more preferably 1 to 3, and still more preferably 1 or 2.

In one aspect, the ring structure may include the following structure.

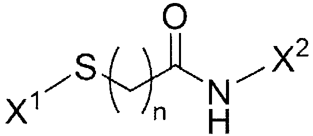

In the formula, X¹ and X² each independently represent any linking group, and n represents an integer of 1 to 10.

The above formula may be a binding site between two amino acid residues in a ring structure. In this case, X¹ and X² are each linked to an amino acid residue, and the amino acid residue linked to X¹ and the amino acid residue linked to X² are linked to each other via one or more other amino acid residues to form a ring structure as a whole.

Examples of X¹ include a single bond and a divalent organic group. Examples of the divalent organic group include a linear or branched alkylene group having 1 to 10 carbon atoms. The number of carbon atoms in the alkylene group having 1 to 10 carbon atoms may be 1 to 5 or may be 1 to 3.

Examples of X² include a single bond and a divalent organic group. Examples of the divalent organic group include a linear or branched alkylene group having 1 to 10 carbon atoms. The number of carbon atoms in the alkylene group having 1 to 10 carbon atoms may be 1 to 5.

n is 1 to 10, preferably 1 to 5, more preferably 1 to 3, and still more preferably 1.

As an example, the structure represented by the formula above is formed by a reaction between an amino acid residue having a thiol group and an amino acid residue having a chloroacetylamino group. In this case, n is 1. In a case where the amino acid residue having a thiol group is a cysteine residue, X¹ is a linear alkylene group having one carbon atom, and in a case where the amino acid residue having a thiol group is a homocysteine residue, X¹ is a linear alkylene group having two carbon atoms. In a case where the chloroacetyl group is bonded to the side chain of the lysine residue, X² represents a linear alkylene group having four carbon atoms, and in a case where the chloroacetyl group is bonded to the main chain of the lysine residue, X² is a single bond.

In one aspect, the polypeptide may be a cyclic polypeptide containing an unnatural amino acid residue. Examples of the cyclic polypeptide containing an unnatural amino acid residue include a polypeptide containing, in one molecule, an amino acid having a thiol group (for example, cysteine) and an amino acid having a chloroacetyl group (for example, chloroacetyl diaminobutyric acid or chloroacetylated lysine). In such a polypeptide, the thiol group reacts with the chloroacetyl group to undergo cyclization, resulting in a cyclic polypeptide.

From the viewpoint of expressing the polypeptide activity, the number of amino acid residues of the polypeptide to be expressed is preferably 6 or more. From the viewpoint of expression efficiency, the number of amino acid residues of the polypeptide is preferably 600 or less, more preferably 300 or less, and still more preferably 150 or less. From such viewpoints, the number of amino acid residues of the polypeptide to be expressed is preferably 6 to 600, more preferably 6 to 300, and still more preferably 6 to 150.

### -Method of cell-free expression-

The cell-free expression of the polypeptide can be performed by a method of performing translation of a template nucleic acid or a method of performing transcription and translation of a template nucleic acid. For the cell-free expression, a cell extract obtained by purifying a cell lysate as necessary may be used, or a reconstituted solution obtained by mixing ribosomes or various other factors prepared by genetic engineering may be used. It is preferable to use a reconstituted solution since it is possible to set conditions under which the introduction efficiency of the unnatural amino acid is high due to the fact that the concentration of each factor can be controlled, and the performance difference between lots is small.

Examples of the cell extract include an Escherichia coli extract, a wheat germ extract, a rabbit erythrocyte extract, and an insect cell extract. The cell extract generally contains various factors such as a ribosome and an initiation factor, which are used for synthesizing a peptide, and various enzyme groups such as tRNA. In a case of performing expression using a cell extract, various amino acids, energy sources such as ATP or GTP, and other substances used in cell-free expression, such as creatine phosphate are generally added to the extract. In addition, a separately prepared ribosome, various factors, various enzyme groups, or the like may be added as necessary.

The reconstituted solution can be constructed using individually purified ribosomal proteins, an aminoacyl-tRNA synthetase (ARS), ribosomal RNA, amino acids, GTP, ATP, an initiation factor (IF), an elongation factor (EF), a release factor (RF), a ribosome regeneration factor, other factors necessary for cell-free peptide synthesis, and the like.

The template nucleic acid may be DNA or mRNA. The template nucleic acid may be a single-stranded nucleic acid or a double-stranded nucleic acid. The template nucleic acid may be a linear nucleic acid or a circular nucleic acid. The template nucleic acid may be a nucleic acid in which a nucleotide sequence required for polypeptide synthesis is incorporated into a vector (a plasmid vector, a cosmid vector, or the like).

From the viewpoint of enabling rapid and low-cost preparation by using primer extension method, a linear DNA is preferable as the template nucleic acid.

The template nucleic acid has a nucleotide sequence necessary for synthesizing a polypeptide by cell-free expression. The template nucleic acid includes a promoter sequence, a sequence encoding a ribosome binding site, a start codon, a sequence encoding an affinity tag, a sequence encoding a polypeptide to be evaluated, and the like.

For example, in the case of the cell-free expression using a factor of Escherichia coli, a promoter that functions in Escherichia coli, for example, a T7 promoter, a lac promoter, or the like can be used to enable expression of the target polypeptide in Escherichia coli. In addition, various promoters can be arranged upstream of the sequence encoding the ribosome binding site. The ribosome binding site is located upstream of the start codon and includes a sequence (SD sequence) to which a ribosome is bound. The SD sequence is a sequence rich in adenine and guanine, and consists of, for example, a sequence of AGGAGG.

In one aspect, the template nucleic acid preferably includes a nucleic acid sequence encoding a translation-promoting sequence immediately after the start codon. The details of the translation-promoting sequence and the nucleotide sequence encoding the translation-promoting sequence are as described above.

In a case of expressing the polypeptide by a cell-free expression, from the viewpoint of increasing the concentration of the polypeptide to be used for evaluating the physiological activity, it is preferable that the types of codons to be used in the template DNA sequence is optimized according to the host for cell-free expression.

In a case of expressing a polypeptide containing an unnatural amino acid by a cell-free expression, there is no restriction on the tRNA used for introducing an unnatural amino acid, and a tRNA that has been improved to increase the introduction efficiency is preferable. Since the aminoacyl tRNA for introducing an unnatural amino acid is easily inactivated by hydrolysis, from the viewpoint of increasing the concentration of the polypeptide to be used for evaluating the physiological activity, the concentration of the aminoacyl tRNA to be added is preferably high, and it is preferable to add the aminoacyl tRNA repeatedly in multiple portions. In addition, from the viewpoint of increasing the concentration of the polypeptide to be used for evaluating the physiological activity, it is preferable to add an aminoacylation enzyme or an artificial enzyme having a similar function to the solution to be used for the cell-free expression, which enables regeneration of hydrolyzed aminoacyl tRNA.

Any known cell-free expression system can be used for cell-free expression. Examples of commercially available products of the cell-free expression system include PUREfrex (GeneFrontier), PURExpress In Vitro Protein Synthesis Kit (New England BioLabs), S30 T7 High-Yield Protein Expression System (Promega), Human Cell-Free Protein Expression System (Takara Bio Inc.), Rapid Translation System (Roche), Expressway Cell-Free Expression System (Invitrogen), and the like.

### [Step (A): binding step]

In step (A), a crude polypeptide solution after cell-free expression is brought into contact with a carrier to bind a polypeptide having a pH-responsive affinity tag to the carrier. The crude polypeptide solution after the cell-free expression refers to a polypeptide solution that either has not undergone any separation or purification after cell-free expression, or still contains components other than the expressed polypeptide even after partial separation and/or purification.

The carrier is not particularly limited as long as it is a carrier to which the affinity tag is specifically bound, and examples thereof include magnetic beads, agarose beads, and beads or plates made of a resin (for example, polystyrene), which have a functional group to which the affinity tag specifically binds, or onto which a molecule that specifically binds to the affinity tag is immobilized.

The form of the carrier is not particularly limited, and may be a form of beads or a form of a column. From the viewpoint of handling a small amount of solution, magnetic beads are preferable since they can be evaluated at a low cost to handle a small amount of solution.

Examples of the molecule that specifically binds to the affinity tag include low-molecular-weight organic compounds, metal compounds (for example, Ni-NTA), hydrophobic molecules, proteins (for example, Strep-Tactin (registered trademark)), and antibodies (for example, anti-PA tag antibodies, anti-FLAG tag antibodies, anti-HA tag antibodies, and anti-c-Myc antibodies).

### [Step (B): elution step]

In the step (B), the polypeptide bound to the carrier is eluted with an acidic aqueous solution or a basic aqueous solution, each having a concentration of less than 50 mM, to obtain an eluted solution. The concentration of the acidic aqueous solution or the basic aqueous solution to be used for the elution is less than 50 mM, and from the viewpoint of reducing the cytotoxicity due to the salt after neutralization, the concentration is more preferably less than 30 mM and particularly preferably less than 20 mM. From the viewpoint of elution efficiency and viewpoint of increasing the concentration of the polypeptide to be used for evaluating the physiological activity, the concentration of the acidic aqueous solution or basic aqueous solution to be used for elution is preferably 0.1 mM or more, more preferably 1 mM or more, and particularly preferably 10 mM. From such viewpoints, the concentration of the acidic aqueous solution or the basic aqueous solution to be used for the elution is preferably 0.1 mM or more and less than 50 mM, more preferably 1 mM or more and less than 30 mM, and still more preferably 10 mM or more and less than 20 mM.

The type of the acidic aqueous solution is not particularly limited, and an acidic aqueous solution that forms a salt which does not exhibit cytotoxicity after neutralization is preferable, and an acidic solution that forms a salt which is contained in a standard culture medium after neutralization is more preferable. Examples of the salt contained in the standard culture medium include sodium chloride, sodium bicarbonate, disodium hydrogen phosphate, potassium chloride, and the like. An acidic solution that forms sodium chloride, which is a salt contained in the largest amount in a standard culture medium after neutralization, is particularly preferable. Examples of the acidic aqueous solution that forms sodium chloride after neutralization include an aqueous solution of hydrochloric acid. Examples of the other acidic aqueous solutions include an aqueous solution of acetic acid, an aqueous solution of carbonic acid, an aqueous solution of citric acid, an aqueous solution of glycine, an aqueous solution of nitric acid, an aqueous solution of sulfuric acid, and the like.

The type of the basic aqueous solution is not particularly limited, and a basic aqueous solution that forms a salt which does not exhibit cytotoxicity after neutralization is preferable, and a basic aqueous solution that forms a salt which is contained in a standard culture medium after neutralization is more preferable. Examples of the type of salt contained in the standard culture medium are the same as those described above. Among these, a basic aqueous solution that forms sodium chloride, which is a salt contained in the largest amount in a standard culture medium after neutralization, is particularly preferable. Examples of the basic aqueous solution that forms sodium chloride after neutralization include an aqueous solution of sodium hydroxide, an aqueous solution of sodium bicarbonate, and an aqueous solution of sodium carbonate. Examples of the other basic aqueous solutions include an aqueous solution of calcium hydroxide, an aqueous solution of ammonia, and the like.

The acidic aqueous solution or the basic aqueous solution to be used for elution may or may not contain other contaminants (buffering components and the like). From the viewpoint of reducing the influence on the evaluation of the physiological activity, it is preferable that other contaminants be not contained.

In the step (B), either an acidic aqueous solution or a basic aqueous solution may be used for elution, and from the viewpoint of suppressing degradation of the polypeptide to be evaluated, it is preferable to use an acidic aqueous solution.

From the viewpoint of the removal efficiency of the cytotoxic components derived from the crude polypeptide solution after the cell-free expression, it is preferable that the carrier be washed after the step (A) and before the step (B).

From the viewpoints of the removal efficiency of the cytotoxic component and the yield of the peptide, the number of times of washing is preferably 2 to 7 times and more preferably 3 to 5 times.

From the viewpoint of the removal efficiency of the cytotoxic component, it is preferable that a surfactant be added to the solution to be used for washing. On the other hand, in a case where a surfactant is added to the solution to be used for washing, it is preferable to perform washing with pure water immediately before elution to reduce the cytotoxicity of the surfactant. The type of the surfactant is not limited, and from the viewpoint of reducing cytotoxicity, a nonionic surfactant is preferable, Tween 20, Pluronic (registered trademark) F-68, Pluronic (registered trademark) F-127, or NP-40 is more preferable, and Tween 20 is still more preferable. The concentration of the surfactant is not limited, and from the viewpoints of cytotoxicity and the removal efficiency of the cytotoxic component, it is preferably 0.1% by mass to 0.0001% by mass and more preferably 0.01% by mass to 0.001% by mass.

### [Step (C): neutralization step]

In the step (C), a basic aqueous solution or an acidic aqueous solution is added to the eluted solution obtained in the step (B) to neutralize the acid or base contained in the eluted solution, and the neutralization is performed in such a manner that the concentration of the salt formed by the neutralization reaction is less than 50 mM, thereby obtaining a polypeptide solution. In a case where an acidic aqueous solution is used for the elution in the step (B), the eluted solution is neutralized with a basic aqueous solution, and in a case where a basic aqueous solution is used for the elution in the step (B), the eluted solution is neutralized with an acidic aqueous solution. In the present disclosure, the term "neutralization" refers to the generation of a salt by a reaction between an acid and a base, and typically refers to neutralization using an equivalent amount of acid and base. However, a case where an excessive amount of an acid or a base is mixed is also not excluded as long as the cytotoxicity does not affect the evaluation of the physiological activity (for example, within a range that does not cause the death of more than 80% or more, preferably 50% or more, and more preferably 10% or more of the viable cells).

From the viewpoint of controlling the pH of the solution after neutralization, the aqueous solution to be used for neutralization is preferably a weakly basic solution rather than a strongly basic solution, and preferably a weakly acidic solution rather than a strongly acidic solution. This is because the pH variation in a case where the mixing ratio deviates from the intended ratio is smaller. As the weakly basic aqueous solution, an aqueous solution of sodium bicarbonate, an aqueous solution of sodium carbonate, or a mixed aqueous solution of sodium bicarbonate and sodium carbonate, which forms sodium chloride, which is a salt contained in the largest amount in a standard culture medium after neutralization, is more preferable. As the weakly acidic aqueous solution, an aqueous solution of carbonic acid is more preferable.

The concentration of the acidic aqueous solution or the basic aqueous solution to be used for neutralization is not particularly limited as long as the concentration of the formed salt is less than 50 mM, and may be determined depending on the acid concentration or the base concentration in the eluted solution in the step (B), the amount of the eluted solution, the amount of the aqueous solution to be used for neutralization, and the like. For example, the concentration of the acidic aqueous solution or the basic aqueous solution to be used for neutralization may be 1 mM to 10 M or 10 mM to 1 M.

The salt concentration of the polypeptide solution after neutralization is less than 50 mM, preferably less than 30 mM, and more preferably less than 20 mM. The salt concentration of the polypeptide solution is preferably as low as possible, but may be 1 mM or more, 2 mM or more, or 5 mM or more. From such a viewpoint, the salt concentration of the polypeptide solution after neutralization may be 1 mM or more and less than 50 mM, 2 mM or more and less than 30 mM, or 5 mM or more and less than 20 mM.

The pH of the polypeptide solution after neutralization is preferably 6 to 9, and more preferably 7 to 8, from the viewpoint of reducing cytotoxicity.

### [Step (D): evaluation step]

In the step (D), the polypeptide solution obtained in the step (C) is brought into contact with cells to evaluate the physiological activity of the polypeptide.

There is no limitation on the type of cell to be used for the evaluation of the physiological activity, and from the viewpoint of sensitivity, specificity, and accuracy of the evaluation, it is preferable to use a cell line that has been improved for the evaluation. Examples of the cell line improved for the evaluation include a cell in which a specific receptor is forcibly expressed, a cell into which a reporter gene has been introduced, and the like.

As a measure for increasing the evaluation concentration of the polypeptide, a method of preparing a culture medium by mixing a large amount of a purified polypeptide solution with a small amount of a concentrated culture medium is considered. Since components composing a commercially available concentrated medium are generally concentrated equally, in a case where the purified polypeptide solution and the concentrated medium are diluted to prepare a culture medium, it is preferable that the salt concentration in the purified polypeptide solution be as low as possible. In a case where the purified polypeptide solution contains a salt, the composition of the prepared culture medium contains a salt at a higher concentration than the appropriate composition, which may cause cytotoxicity.

There is no restriction on the type of the culture medium to be used for evaluating the physiological activity, and it is preferable to select an appropriate culture medium according to the type of the evaluation of the physiological activity and the type of the cells to be used. From the viewpoint of increasing the concentration of the polypeptide to be used for evaluating the physiological activity, it is more preferable to dilute a concentrated culture medium with the polypeptide solution after neutralization and use the resulting mixture.

### [Other steps]

The method of the present disclosure may include additional steps in addition to the cell-free expression, the steps (A) to (D), and the washing step before the step (B). Examples of the additional steps include an adjustment step of DNA to be used for the cell-free expression, a step of quantifying the polypeptide concentration after the cell-free expression or after the step (C), and the like. From the viewpoint of obtaining a high yield of the polypeptide, it is preferable that the method according to the present disclosure does not include another purification step (size exclusion column purification or the like) after the step (C) and before the evaluation of the physiological activity.

### Examples

Hereinafter, the embodiment of the present disclosure will be more specifically described with reference to Examples, but the embodiment of the present disclosure is not limited to the following Examples.

### <Example 1: evaluation of cytotoxicity>

In the present example, in evaluating the physiological activity of the polypeptide synthesized by the cell-free expression method, the cytotoxicity was evaluated for a crude polypeptide solution after cell-free expression, an eluted solution of affinity purification, and a solution obtained by neutralizing the eluted solution of affinity purification. In the present example, to evaluate the cytotoxicity of the reaction system to be used for the cell-free expression, the cell-free expression reaction was performed without adding the template DNA to prepare a cell-free expression solution containing no polypeptide, and the affinity purification procedure was performed on this solution.

Chloroacetylated lysine was selected as an unnatural amino acid, and to introduce chloroacetylated lysine into a polypeptide, a tRNA having an anticodon of CUA that pairs with a UAG codon of mRNA was prepared by transcribing the DNA of SEQ ID NO: 1. The tRNA was aminoacylated with an N-chloroacetylated lysine 5'-phospho-2'-deoxyribocytidylylriboadenosine (pdCpA) ester. The produced aminoacyl tRNA is referred to as aminoacyl tRNA (1).

SEQ ID NO: 1:
GTTGTAAAACGACGGCCAGTGCCAAGCTTGGGCTAATACGACTCACTATAGGGAG
AGTAGTTCAATGGTAGAACGTCGGTCTCTAAAACCGAGCGTTGAGGGTTCGATTC
CTTTCTCTCCCAC

As cell-free expression solutions, reaction solutions were prepared with the following compositions using three types, PUREfrex 2.0 (GeneFrontier Corporation, PF201-0.25-5), PURExpress In Vitro Protein Synthesis Kit (NEW ENGLAND BioLabs, E6800S), and S30T7 High-Yield Protein Expression System (Promega, L1110), and reacted at 37°C for 1 hour to prepare cell-free expression solutions containing no polypeptide.
· PUREfrex 2.0: 5.0 µL of Solution I, 0.5 µL of Solution II, 1.0 µL of Solution III, a dried substance of aminoacyl tRNA (1) (final concentration of 0.5 µg/µL), and 3.5 µL of water were mixed.
· PURExpress In Vitro Protein Synthesis Kit: 4.0 µL of Solution A, 3.0 µL of Solution B, a dried substance of aminoacyl tRNA (1) (final concentration of 0.5 µg/µL), and 3.0 µL of water were mixed.
· S30 T7 High-Yield Protein Expression System: 4.0 µL of S30 Premix Pllus, 3.6 µL of T7 S30 Extract, a dried substance of aminoacyl tRNA (1) (final concentration of 0.5 µg/µL), and 2.4 µL of water were mixed.

The cell-free expression solutions were subjected to affinity purification and a neutralization treatment according to the conditions shown in Table 2.

It is noted that in a case of the condition in which affinity purification was not performed, 6.5 µL of the cell-free expression solution was taken and mixed with 17.5 µL of water to unify the dilution ratio of the cell-free expression solution with the condition used in affinity purification.

The following procedures were performed for the conditions for performing affinity purification.

The following magnetic beads were used as an affinity carrier to be used for affinity purification.
· PA tag: MagCapture HP Anti-PA Tag Antibody Magnetic Beads (FUJIFILM Wako Pure Chemical Corporation, 137-18751)
· FLAG (registered trademark) tag: Anti-DYKDDDDK Tag Antibody Magnetic Beads (FUJIFILM Wako Pure Chemical Corporation, 011-25154)
· HA tag: Pierce Anti-HA Magnetic Beads (Thermo Fisher Scientific, 88836)
· His tag: HisPur Ni-NTA Magnetic Beads (Thermo Fisher Scientific, 88831)
· c-Myc tag: Pierce Anti-c-Myc Magnetic Beads (Thermo Fisher Scientific, 88842)
· Strep tag (registered trademark): MagStrep "type3" Strep-Tactin (registered trademark) Beads (IBA Lifesciences, 2-1613-002)

First, a required amount of beads to be used for affinity purification was taken and washed three times with PBS-T.

Next, 8.0 µL of the cell-free expression solution, 10 µL of the washed beads, and 2.0 µL of PBS-T were mixed, and shaken and mixed at room temperature (approximately 25°C) for 2 hours to perform the binding step. Thereafter, the mixture was placed on a magnet stand, and the supernatant was removed, followed by three washes with 100 µL of washing solution each. The types of cleaning liquids used are as shown in Table 2.

Next, the beads after washing were placed on a magnet stand, the supernatant was removed, 25 µL of the liquid A was added thereto, and the mixture was shaken and mixed at room temperature for 15 minutes to perform the elution step.

The eluted solution was placed on a magnet stand, and 21.6 µL of the supernatant of the eluted solution was collected, and mixed with 2.4 µL of the liquid B to perform the neutralization step. The types of the liquids A and B used are as shown in Table 2, and the liquids specifically used are as follows.
· Hydrochloric acid: 0.1 mol/L hydrochloric acid (FUJIFILM Wako Pure Chemical Corporation, 081-01111) was prepared in water at the concentration shown in Table 2 and used.
· PA peptide: PA tag peptide (FUJIFILM Wako Pure Chemical Corporation, 161-28681) was prepared in water at the concentration shown in Table 2 and used.
· NaOH: 0.1 mol/L sodium hydroxide (FUJIFILM Wako Pure Chemical Corporation, 194-02191) was prepared in water at the concentration shown in Table 2 and used.
· Sodium bicarbonate: sodium bicarbonate (KANTO CHEMICAL CO., INC., 37116-23) was prepared in water to have a concentration shown in Table 2 and used.

As a comparative example in which contaminants were removed after the elution step, for the conditions for size exclusion column purification of the eluted solution, 75 µL spin column was filled with Pierce^{™} Polyacrylamide Desalting Columns (Thermo Fisher Scientific, 43426) and the size exclusion column purification was performed according to the manufacturer-recommended protocol.

The cytotoxicity of each solution was evaluated using BaF3 cells stably expressing fibroblast growth factor receptor (FGFR). The present cells have properties of proliferating in the presence of various fibroblast growth factors (FGF) and analogous active molecules and dying in the absence thereof. BaF3 cells stably having expressed FGFR were established by introducing a vector (Gene Copoeia, EX-Y2820-M67) incorporating an FGFR gene into BaF3 cells using Nucleofector (Lonza) and selecting cells into which the FGFR gene had been introduced using hygromycin.

To allow the cell-free expression solution to act on cells at a lower dilution ratio, an RPMI medium (hereinafter, referred to as a "concentrated medium") 3 times more concentrated than the usual composition was prepared by mixing 750 µL of RPMI-1640 (10×) (Sigma-Aldrich, R1145), 750 µL of Fetal Bovine Serum (Thermo Fisher Scientific, 10270-106), 75 µL of Penicillin Streptomycin (Thermo Fisher Scientific, 15140-122), 75 µL of GlutaMAX (Thermo Fisher Scientific, 35050-061), 200 µL of Sodium Bicarbonate 7.5% solution (Thermo Fisher Scientific, 25080094), 75 µL of folic acid (FUJIFILM Wako Pure Chemical Corporation, 060-01802, dissolved to a concentration of 100 µg/mL in a 1% DMSO aqueous solution), 10 µL of Heparin Solution (2 mg/mL) (STEM CELL, 07980), 40 µL of bFGF (a type of FGF, R&D Systems, 3718-GMP, dissolved to a concentration of 0.25 µM in PBS), and 525 µL of water.

The BaF3 cells stably expressing FGFR were washed twice with PBS, and then prepared to 4 × 10⁵ cells/mL using a culture medium obtained by mixing RPMI-1640 (containing L-glutamine and phenol red) (FUJIFILM Wako Pure Chemical Corporation, 189-02025) and Fetal Bovine Serum (Thermo Fisher Scientific, 10270-106) at a ratio of 10:1.

A liquid obtained by mixing 24 µL of each solution (a crude polypeptide solution after cell-free expression, an eluted solution after affinity purification, or a solution obtained by neutralizing the eluted solution after affinity purification) with 12 µL of a concentrated culture medium was dispensed in 15 µL portions into a 384-well plate (Perkin Elmer, 6057300), 5 µL of the above-described cell suspension was then added to each well, and the mixture was shaken and mixed, and then cultured at 37°C (5% CO₂) for 2 nights. In this case, as a positive control having no cytotoxicity, wells were prepared in which 15 µL of a liquid obtained by mixing 24 µL of water with 12 µL of the concentrated culture medium was dispensed instead of each liquid.

After culturing for 2 nights, Hoechst 33342, Trihydrochloride, Trihydrate - 10 mg/mL Solution in Water (Thermo Fisher Scientific, H3570), -Cellstain (registered trademark) - Calcein-AM solution (1 mg/mL DMSO solution) (DOJINDO, C396), and -Cellstain (registered trademark) - PI solution (DOJINDO, P378) were each added to the RPMI (10% FBS) medium at a final concentration of 1/1000 by volume, and Live/dead staining was performed by allowing the mixture to stand at room temperature for 1 hour, and imaging was performed using a confocal microscope (Yokogawa Electric Corporation, CQ1). The number of viable cells was calculated from the imaging data, and the number of cells in each well was normalized to 100 based on the number of viable cells present in the well of the positive control. The results are shown in Table 2.

The evaluation standards for the cytotoxicity are as follows. Each well was determined as Evaluation A to D according to the number of cells in the well, normalized to 100 based on the number of viable cells present in the well of the positive control. It is preferable that the evaluation is A, B, or C.
A: 90 or more
B: 50 or more and less than 90
C: 20 or more and less than 50
D: less than 20

**[Table 2]**

| Conditions of affinity purification and neutralization treatment for cell-free expression solution and results of cytotoxicity evaluation | | | | | | | | |
|---|---|---|---|---|---|---|---|---|
| No. | Cell-free expression solution | Purification | Affinity tag | Washing step | Liquid A | Liquid B | Evaluation grade | Remarks |
| 1 | PUREfrex | No | - | - | - | - | D | |
| 2 | PUREfrex | Yes | PA | 2 times with PBS-T → 1 time with water | 10 mM hydrochloric acid | 100 mM sodium bicarbonate | A | |
| 3 | PUREfrex | Yes | PA | 2 times with PBS-T → 1 time with water | 20 mM hydrochloric acid | 200 mM sodium bicarbonate | B | |
| 4 | PUREfrex | Yes | PA | 2 times with PBS-T → 1 time with water | 30 mM hydrochloric acid | 300 mM sodium bicarbonate | C | |
| 5 | PUREfrex | Yes | PA | 2 times with PBS-T → 1 time with water | 40 mM hydrochloric acid | 400 mM sodium bicarbonate | C | |
| 6 | PUREfrex | Yes | PA | 2 times with PBS-T → 1 time with water | 50 mM hydrochloric acid | 500 mM sodium bicarbonate | D | |
| 7 | PUREfrex | Yes | PA | 2 times with PBS-T → 1 time with water | 60 mM hydrochloric acid | 600 mM sodium bicarbonate | D | |
| 8 | PUREfrex | Yes | PA | 2 times with PBS-T → 1 time with water | 80 mM hydrochloric acid | 800 mM sodium bicarbonate | D | |
| 9 | PUREfrex | Yes | PA | 2 times with PBS-T → 1 time with water | 100 mM hydrochloric acid | 1000 mM sodium bicarbonate | D | |
| 10 | PUREfrex | Yes | PA | 2 times with PBS-T → 1 time with water | 30 mM hydrochloric acid | Water | D | |
| 11 | PUREfrex | Yes | PA | 3 times with PBS-T | 10 mM hydrochloric acid | 100 mM sodium bicarbonate | B | |
| 12 | PUREfrex | Yes | PA | 2 times with PBS-T → 1 time with water | 0.5 mg/mL PA peptide | Water | B | |
| 13 | PUREfrex | Yes | FLAG | 2 times with PBS-T → 1 time with water | 100 mM hydrochloric acid | 100 mM sodium bicarbonate | A | |
| 14 | PUREfrex | Yes | HA | 2 times with PBS-T → 1 time with water | 100 mM hydrochloric acid | 100 mM sodium bicarbonate | A | |
| 15 | PUREfrex | Yes | His | 2 times with PBS-T → 1 time with water | 100 mM hydrochloric acid | 100 mM sodium bicarbonate | A | |
| 22 | PUREfrex | Yes | Strep | 2 times with PBS-T → 1 time with water | 100 mM hydrochloric acid | 100 mM sodium bicarbonate | A | |
| 23 | PUREfrex | Yes | c-Myc | 2 times with PBS-T → 1 time with water | 100 mM hydrochloric acid | 100 mM sodium bicarbonate | A | |
| 24 | PUREfrex | Yes | HA | 2 times with PBS-T → 1 time with water | 10 mM sodium hydroxide | 90 mM hydrochloric acid | A | |
| 25 | PUREfrex | Yes | c-Myc | 2 times with PBS-T → 1 time with water | 10 mM sodium hydroxide | 90 mM hydrochloric acid | A | |
| 26 | PUREfrex | Yes | PA | 2 times with PBS-T → 1 time with water | 100 mM hydrochloric acid | 1000 mM sodium bicarbonate | A | Desalting treatment after neutralization |
| 27 | PURExpress | No | - | - | - | - | D | |
| 28 | PURExpress | Yes | PA | 2 times with PBS-T → 1 time with water | 10 mM hydrochloric acid | 100 mM sodium bicarbonate | A | |
| 29 | S30 | No | - | - | - | - | D | |
| 30 | S30 | Yes | PA | 2 times with PBS-T → 1 time with water | 10 mM hydrochloric acid | 100 mM sodium bicarbonate | C | |

Under the conditions (Nos. 1, 27, and 29) in which affinity purification has never been performed, the cells are completely dead due to the toxicity of the cell-free expression solution, and under these conditions, it is impossible to evaluate the physiological activity.

Even under the conditions in which affinity purification has been performed, the number of viable cells is significantly reduced due to the cytotoxicity of the mixed solution of the liquid A and the liquid B under the conditions in which the concentration of the dissolved substance of the liquid A or the liquid B is high (Nos. 6 to 9).

In addition, under the condition in which the neutralization by the liquid B has never been performed (No. 10), the number of viable cells is significantly reduced due to the pH of the liquid A. In this way, it is extremely difficult to evaluate the physiological activity under the near-lethal condition of the cell.

Reduction o the number of viable cells is observed under the condition (No. 12) in which the liquid A is the PA peptide solution. Under such conditions, it is not impossible to perform the evaluation of the physiological activity, but as described later in Example 2, the method of competitively eluting a target substance using a peptide in this manner has a low elution efficiency and a significantly reduced evaluation concentration, whereby it is difficult to use the method for the evaluation of the physiological activity. In a case where the concentration of the PA peptide is increased to improve the elution efficiency, the toxicity also becomes strong due to the presence of the PA peptide at a high concentration, whereby it is difficult to achieve both high elution efficiency and low toxicity. Therefore, it is extremely difficult to evaluate the physiological activity using competitive elution.

The number of viable cells is maintained under the condition (No. 26) in which the size exclusion column purification step has been performed to remove the contaminant components after the elution, but as described later in Example 2, the peptide loss in the step of removing the contaminant components is large, and it is difficult to achieve both a high evaluation concentration and low toxicity.

The conditions (Nos. 2 to 5, 11, 13 to 25, 28, and 30) in which the liquids A and B are a low concentration of an acid or a base indicates that the number of the viable cells is maintained, and evaluation of the physiological activity can be performed under this condition.

In a case of comparing No. 2, in which the washing step immediately before the elution uses water, with No. 11, in which the washing step immediately before the elution uses PBS-T, the number of viable cells is larger in No. 2. Therefore, it is shown that the toxicity of PBS-T, which is slightly mixed, can be removed by washing with water immediately before the elution, and the decrease in the number of viable cells can be further suppressed. From this result, it is possible to further improve the accuracy and sensitivity of the evaluation of the physiological activity by washing with water immediately before the elution.

### <Example 2: Evaluation of physiological activity of affinity-purified polypeptide>

In the present example, after performing the cell-free expression of the polypeptide of SEQ ID NO: 2 having a bFGF-like physiological activity, affinity purification and neutralization treatment were performed, and the yield in the affinity purification and neutralization treatment and the physiological activity using the polypeptide solution obtained by performing the affinity purification and neutralization treatment were evaluated. The yield in the affinity purification and the neutralization treatment is an important performance index for achieving the evaluation of the physiological activity at a high polypeptide concentration. 
SEQ ID NO: 2: H-C-SYERLQFHGHEAPFRV-X-VSH-C-SYERLQFHGHEAPFRV-
X-V
 (X represents a chloroacetylated lysine residue, which is an unnatural amino acid residue. The present polypeptide is a polypeptide having two rings, in which a thioether bond is formed between a side chain of a first cysteine and a side chain of a first chloroacetylated lysine from the N-terminus, and another thioether bond is also formed between a side chain of a second cysteine and a side chain of a second chloroacetylated lysine from the N-terminus.)

The cell-free expression of the polypeptide was performed according to the following procedure.

First, fusion peptides (SEQ ID NOs: 11 to 16) were designed in which a translation-promoting sequence (SEQ ID NO: 3), a polypeptide of SEQ ID NO: 2, a HiBiT tag for concentration quantification (SEQ ID NO: 4), and an affinity tag for affinity purification (any one of SEQ ID NOs: 5 to 10) were linked in order via a GGS linker.
SEQ ID NO: 3: VKKTKT
SEQ ID NO: 4: VSGWRLFKKIS
SEQ ID NO: 5 (PA tag): GVAMPGAEDDVV
SEQ ID NO: 6 (FLAG tag): DYKDDDDK
SEQ ID NO: 7 (HA tag): YPYDVPDYA
SEQ ID NO: 8 (His tag): HHHHHH
SEQ ID NO: 9 (c-Myc tag): EQKLISEEDL
SEQ ID NO: 10 (Strep tag): WSHPQFEK
SEQ ID NO: 11 (fusion peptide with PA tag): MVKKTKTH-C-SYERLQFHGHEAPFRV-X-VSH-C-SYERLQFHGHEAPFRV-X-VGSGVSGWRLFKKISSGSGVAMPGAEDDVV
SEQ ID NO: 12 (fusion peptide with FLAG tag): MVKKTKTH-C-SYERLQFHGHEAPFRV-X-VSH-C-SYERLQFHGHEAPFRV-X-VGSGVSGWRLFKKISSGSDYKDDDDK
SEQ ID NO: 13 (fusion peptide with HA tag): MVKKTKTH-C-SYERLQFHGHEAPFRV-X-VSH-C-SYERLQFHGHEAPFRV-X-VGSGVSGWRLFKKISSGSYPYDVPDYA
SEQ ID NO: 14 (fusion peptide with His tag): MVKKTKTH-C-SYERLQFHGHEAPFRV-X-VSH-C-SYERLQFHGHEAPFRV-X-VGSGVSGWRLFKKISSGSHHHHHH
SEQ ID NO: 15 (fusion peptide with c-Myc tag): MVKKTKTH-C-SYERLQFHGHEAPFRV-X-VSH-C-SYERLQFHGHEAPFRV-X-VGSGVSGWRLFKKISSGSEQKLISEEDL
SEQ ID NO: 16 (fusion peptide with Strep tag): MVKKTKTH-C-SYERLQFHGHEAPFRV-X-VSH-C-SYERLQFHGHEAPFRV-X-VGSGVSGWRLFKKISSGSWSHPQFEK

Next, template DNA sequences (SEQ ID NOs: 17 to 22) were designed in which sequences necessary for transcription and translation initiation, such as a T7 promoter sequence, a Shine-Dalgarno sequence, and a start codon (ATG), were provided on the 5'-side of the DNA sequence encoding the above-described fusion peptides (SEQ ID NOs: 11 to 16), and a stop codon group was provided on the 3'-side thereof. In a case where the triplet TAG codon is included in the template DNA sequence, the above-described codon is assigned to the codon of chloroacetylated lysine, and thus, the peptide encoded by the above-described DNA sequence is a polypeptide having two rings, in which a thiol group of cysteine and a chloroacetyl group of chloroacetylated lysine spontaneously form a thioether bond.
SEQ ID NO: 17 (template DNA for fusion peptide with PA tag): 
gaaattaatacgactcactatagggagaccacaacggtttccctctagaaataattttgtttaactttaagaaggagatataccaATGGT
TAAAAAAACAAAAACACATTGCTCTTACGAACGTCTGCAGTTCCATGGTCATGAA
GCTCCGTTCCGTGTTTAGGTTTCGCACTGTAGCTATGAGCGCTTGCAATTTCACGG
CCACGAGGCACCCTTTCGCGTATAGGTAGGAAGTGGTGTAAGTGGATGGCGATTA
 
TTCAAGAAGATTAGCAGCGGCAGTGGAGTTGCAATGCCTGGTGCAGAAGATGATG
TAGTAtaatga
SEQ ID NO: 18 (template DNA for fusion peptide with FLAG tag): 
gaaattaatacgactcactatagggagaccacaacggtttccctctagaaataattttgtttaactttaagaaggagatataccaATG
GTTAAAAAAACAAAAACACATTGCTCTTACGAACGTCTGCAGTTCCATGGTCATG
AAGCTCCGTTCCGTGTTTAGGTTTCGCACTGTAGCTATGAGCGCTTGCAATTTCAC
GGCCACGAGGCACCCTTTCGCGTATAGGTAGGAAGTGGTGTAAGTGGATGGCGAT
TATTCAAGAAGATTAGCAGCGGCAGTGACTACAAAGACGACGACGACAAAtaatga
SEQ ID NO: 19 (template DNA for fusion peptide with HA tag): 
gaaattaatacgactcactatagggagaccacaacggtttccctctagaaataattttgtttaactttaagaaggagatataccaATGGT
TAAAAAAACAAAAACACATTGCTCTTACGAACGTCTGCAGTTCCATGGTCATGAA
GCTCCGTTCCGTGTTTAGGTTTCGCACTGTAGCTATGAGCGCTTGCAATTTCACGG
CCACGAGGCACCCTTTCGCGTATAGGTAGGAAGTGGTGTAAGTGGATGGCGATTA
TTCAAGAAGATTAGCAGCGGCAGTTACCCGTACGACGTTCCGGACTACGCTtaatga
SEQ ID NO: 20 (DNA template for His-tagged fused peptide): 
gaaattaatacgactcactatagggagaccacaacggtttccctctagaaataattttgtttaactttaagaaggagatataccaATGGT
TAAAAAAACAAAAACACATTGCTCTTACGAACGTCTGCAGTTCCATGGTCATGAA
GCTCCGTTCCGTGTTTAGGTTTCGCACTGTAGCTATGAGCGCTTGCAATTTCACGG
CCACGAGGCACCCTTTCGCGTATAGGTAGGAAGTGGTGTAAGTGGATGGCGATTA
TTCAAGAAGATTAGCAGCGGCAGTCATCATCATCATCATCATtaatga
SEQ ID NO: 21 (template DNA for fusion peptide with c-Myc tag): 
gaaattaatacgactcactatagggagaccacaacggtttccctctagaaataattttgtttaactttaagaaggagatataccaATGGT
TAAAAAAACAAAAACACATTGCTCTTACGAACGTCTGCAGTTCCATGGTCATGAA
GCTCCGTTCCGTGTTTAGGTTTCGCACTGTAGCTATGAGCGCTTGCAATTTCACGG
CCACGAGGCACCCTTTCGCGTATAGGTAGGAAGTGGTGTAAGTGGATGGCGATTA
TTCAAGAAGATTAGCAGCGGCAGTGAACAGAAACTGATCTCTGAAGAAGACCTGt
aatga
SEQ ID NO: 22 (template DNA for fusion peptide with Strep tag):

gaaattaatacgactcactatagggagaccacaacggtttccctctagaaataattttgtttaactttaagaaggagatataccaATGGT
TAAAAAAACAAAAACACATTGCTCTTACGAACGTCTGCAGTTCCATGGTCATGAA
GCTCCGTTCCGTGTTTAGGTTTCGCACTGTAGCTATGAGCGCTTGCAATTTCACGG
CCACGAGGCACCCTTTCGCGTATAGGTAGGAAGTGGTGTAAGTGGATGGCGATTA
TTCAAGAAGATTAGCAGCGGCAGTTGGTCTCATCCGCAGTTCGAAAAAtaatga

In the above-described sequences, a DNA sequence encoding a fusion peptide is described in capital letters, and sequences added to the 5'-end and the 3'-end are described in small letters. In addition, a line is drawn under the DNA sequence encoding the affinity tag for purification.

The template DNA was produced by a two-step overlap extension PCR.

In the first stage of PCR, the DNA of SEQ ID NO: 25 and the DNA of SEQ ID NO: 26 were linked by mixing four types of DNA, the DNA of SEQ ID NO: 23, the DNA of SEQ ID NO: 24, the DNA of SEQ ID NO: 25, and the DNA of SEQ ID NO: 26, such that the concentrations thereof were 0.3 µmol/L, 0.3 µmol/L, 0.05 µmol/L, and 0.05 µmol/L, and in the presence of PrimeSTAR Max (TaKaRa, R045B), after performing a step of 98°C/10 seconds, 39°C/5 seconds, and 72°C/5 seconds, repeatedly performing three steps of 98°C/10 seconds, 58°C/5 seconds, and 72°C/5 seconds for 27 cycles. The linked DNA was purified and diluted to 50 ng/µL.
SEQ ID NO: 23: GAAATTAATACGACTCACTATAGG
SEQ ID NO: 24: CGAAACCTAAACACGGAA
SEQ ID NO: 25: 
GAAATTAATACGACTCACTATAGGGAGACCACAACGGTTTCCCTCTAGAAATAAT
TTTGTTTAACTTTAAGAAGGAGATATACCAATGGTTAAAAAAACAAAAAC
SEQ ID NO: 26:

CGAAACCTAAACACGGAACGGAGCTTCATGACCATGGAACTGCAGACGTTCGTAA
GAGCAATGTGTTTTTGTTTTTTTAAC

In the second stage of PCR, by mixing four types of DNA, the DNA of SEQ ID NO: 23, any one of the DNAs of SEQ ID NOs: 27 to 32, any one of the DNAs of SEQ ID NOs: 33 to 38, and the purified product of the first stage, such that the concentrations thereof were 0.3 µmol/L, 0.3 µmol/L, 0.0025 µmol/L, and 0.4 ng/µL, and in the presence of PrimeSTAR Max (TaKaRa, R045B), and repeatedly performing three steps of 98°C/10 seconds, 58°C/5 seconds, and 72°C/5 seconds for 30 cycles, the DNA of SEQ ID NOs: 33 to 38 and the purified product of the first stage were linked to obtain the template DNA (SEQ ID NOs: 17 to 22). In this case, the DNA of SEQ ID NOs: 27 to 32 and the DNA of SEQ ID NOs: 33 to 38 were combined with the same tag type to perform the linking reaction. The obtained template DNA was purified and diluted to 50 ng/µL.
SEQ ID NO: 27 (PA tag): TCATTATACTACATCATCTTCTGCACCAG
SEQ ID NO: 28 (FLAG tag): TCATTATTTGTCGTCGTCG
SEQ ID NO: 29 (HA tag): TCATTAAGCGTAGTCCGGAAC
SEQ ID NO: 30 (His tag): TCATTAATGATGATGATGATGA
SEQ ID NO: 31 (c-Myc tag): TCATTACAGGTCTTCTTCAGAGATCAG
SEQ ID NO: 32 (Strep tag): TCATTATTTTTCGAACTGCG
SEQ ID NO: 33 (PA tag): 
CATCATCTTCTGCACCAGGCATTGCAACTCCACTGCCGCTGCTAATCTTCTTGAAT
AATCGCCATCCACTTACACCACTTCCTACCTATACGCGAAAGGGTGCCTCGTGGCC
GTGAAATTGCAAGCGCTCATAGCTACAGTGCGAAACCTAAACACGGAA
SEQ ID NO: 34 (FLAG tag): 
TCATTATTTGTCGTCGTCGTCTTTGTAGTCACTGCCGCTGCTAATCTTCTTGAATAA
TCGCCATCCACTTACACCACTTCCTACCTATACGCGAAAGGGTGCCTCGTGGCCGT
GAAATTGCAAGCGCTCATAGCTACAGTGCGAAACCTAAACACGGAA
SEQ ID NO: 35 (HA tag): 
ATTAAGCGTAGTCCGGAACGTCGTACGGGTAACTGCCGCTGCTAATCTTCTTGAAT
AATCGCCATCCACTTACACCACTTCCTACCTATACGCGAAAGGGTGCCTCGTGGCC
GTGAAATTGCAAGCGCTCATAGCTACAGTGCGAAACCTAAACACGGAA
SEQ ID NO: 36 (His tag): 
TCATTAATGATGATGATGATGATGACTGCCGCTGCTAATCTTCTTGAATAATCGCC
ATCCACTTACACCACTTCCTACCTATACGCGAAAGGGTGCCTCGTGGCCGTGAAAT
TGCAAGCGCTCATAGCTACAGTGCGAAACCTAAACACGGAA
SEQ ID NO: 37 (c-Myc tag): 
ACAGGTCTTCTTCAGAGATCAGTTTCTGTTCACTGCCGCTGCTAATCTTCTTGAAT
AATCGCCATCCACTTACACCACTTCCTACCTATACGCGAAAGGGTGCCTCGTGGCC
GTGAAATTGCAAGCGCTCATAGCTACAGTGCGAAACCTAAACACGGAA
SEQ ID NO: 38 (Strep tag):

TCATTATTTTTCGAACTGCGGATGAGACCAACTGCCGCTGCTAATCTTCTTGAATA
ATCGCCATCCACTTACACCACTTCCTACCTATACGCGAAAGGGTGCCTCGTGGCCG
TGAAATTGCAAGCGCTCATAGCTACAGTGCGAAACCTAAACACGGAA

The produced template DNA was subjected to cell-free expression, affinity purification, and neutralization treatment according to the conditions shown in Table 3.

The cell-free expression of the polypeptide was performed, in the same manner as in Example 1, using three types, PUREfrex 2.0 (GeneFrontier Corporation, PF201-0.25-5), PURExpress In Vitro Protein Synthesis Kit (NEW ENGLAND BioLabs, E6800S), and S30T7 High-Yield Protein Expression System (Promega, L1110). Each reaction solution was prepared with the following composition, and the reaction was performed at 37°C for 1 hour to perform the cell-free expression of the polypeptide.

· PUREfrex 2.0: 5.0 µL of Solution I, 0.5 µL of Solution II, 1.0 µL of Solution III, a dried substance of aminoacyl tRNA (1) (final concentration of 0.5 µg/µL), 2.0 µL of template DNA, and 1.5 µL of water were mixed.
· PURExpress In Vitro Protein Synthesis Kit: 4.0 µL of Solution A, 3.0 µL of Solution B, a dried substance of aminoacyl tRNA (1) (final concentration of 0.5 µg/µL), 2.0 µL of template DNA, and 1.0 µL of water were mixed.
· S30 T7 High-Yield Protein Expression System: 4.0 µL of S30 Premix Pllus, 3.6 µL of T7 S30 Extract, a dried substance of aminoacyl tRNA (1) (final concentration of 0.5 µg/µL), 2.0 µL of template DNA, and 0.4 µL of water were mixed.

The polypeptide produced by cell-free expression was subjected to affinity purification and neutralization treatment in the same manner as in Example 1. In a case of the condition in which affinity purification was not performed, in the same manner as in Example 1, 6.5 µL of the cell-free expression solution was taken and mixed with 17.5 µL of water to unify the dilution ratio of the cell-free expression solution with the condition used in affinity purification.

The polypeptide concentration after affinity purification and neutralization treatment was measured according to the standard protocol of the Nano Glo HiBiT Lytic Detection System using the affinity-purified and neutralized product obtained by 100-fold dilution with Can Get Signal Immunoreaction Enhancer Solution I (TOYOBO, NKB-101), Nano Glo HiBiT Lytic Detection System (Promega, N3040), and a peptide (SEQ ID NO: 39) having a known concentration. The obtained polypeptide concentration is shown in Table 3 to two significant figures. The peptide of SEQ ID NO: 39 was produced by chemical synthesis, then dissolved in PBS (-) to a concentration of 500 µM, and used after appropriate dilution of the solution.
SEQ ID NO: 39: EQKLISEEDLGGSVSGWRLFKKIS

The polypeptide yield in affinity purification and neutralization treatment was calculated based on the polypeptide concentration under the condition in which affinity purification was not performed, and the results are shown in Table 3.

The evaluation standards for the polypeptide yield in the affinity purification and the neutralization treatment are as follows. The polypeptide was determined as Evaluation A to D according to the yield in the affinity purification and the neutralization treatment, as defined by the following percentages. It is preferable that the evaluation is A, B, or C.
A: 50% or more
B: 10% or more and less than 50%
C: 5% or more and less than 10%
D: less than 5%

**[Table 3]**

| Polypeptide yield in affinity purification and neutralization treatment | | | | | | | | |
|---|---|---|---|---|---|---|---|---|
| No. | Cell-free expression solution | Purification | Affinity tag | Washing step | Liquid A | Liquid B | Evaluation grade | Remarks |
| 2 | PUREfrex | Yes | PA | 2 times with PBS-T → 1 time with water | 10 mM hydrochloric acid | 100 mM sodium bicarbonate | A | |
| 3 | PUREfrex | Yes | PA | 2 times with PBS-T → 1 time with water | 20 mM hydrochloric acid | 200 mM sodium bicarbonate | A | |
| 4 | PUREfrex | Yes | PA | 2 times with PBS-T → 1 time with water | 30 mM hydrochloric acid | 300 mM sodium bicarbonate | A | |
| 5 | PUREfrex | Yes | PA | 2 times with PBS-T → 1 time with water | 40 mM hydrochloric acid | 400 mM sodium bicarbonate | A | |
| 11 | PUREfrex | Yes | PA | 3 times with PBS-T | 10 mM hvdrochloric acid | 100 mM sodium bicarbonate | A | |
| 12 | PUREfrex | Yes | PA | 2 times with PBS-T → 1 time with water | 0.5 mg/mL PA peptide | Water | D | |
| 13 | PUREfrex | Yes | FLAG | 2 times with PBS-T → 1 time with water | 10 mM hydrochloric acid | 100 mM sodium bicarbonate | B | |
| 14 | PUREfrex | Yes | HA | 2 times with PBS-T → 1 time with water | 10 mM hydrochloric acid | 100 mM sodium bicarbonate | A | |
| 15 | PUREfrex | Yes | His | 2 times with PBS-T → 1 time with water | 10 mM hydrochloric acid | 100 mM sodium bicarbonate | A | |
| 22 | PUREfrex | Yes | Strep | 2 times with PBS-T → 1 time with water | 10 mM hydrochloric acid | 100 mM sodium bicarbonate | C | |
| 23 | PUREfrex | Yes | c-Myc | 2 times with PBS-T → 1 time with water | 10 mM hydrochloric acid | 100 mM sodium bicarbonate | A | |
| 24 | PUREfrex | Yes | HA | 2 times with PBS-T → 1 time with water | 10 mM sodium hydroxide | 90 mM hydrochloric acid | A | |
| 25 | PUREfrex | Yes | c-Myc | 2 times with PBS-T → 1 time with water | 10 mM sodium hydroxide | 90 mM hydrochloric acid | A | |
| 26 | PUREfrex | Yes | PA | 2 times with PBS-T → 1 time with water | 100 mM hydrochloric acid | 1000 mM sodium bicarbonate | D | Desalting treatment after neutralization |
| 28 | PURExpress | Yes | PA | 2 times with PBS-T → 1 time with water | 10 mM hydrochloric acid | 100 mM sodium bicarbonate | A | |
| 30 | S30 | Yes | PA | 2 times with PBS-T → 1 time with water | 10 mM hydrochloric acid | 100 mM sodium bicarbonate | A | |

The yield of the polypeptide slightly differs depending on the type of tag, but in the conditions (Nos. 2 to 5, 11, 13 to 15, 22 to 25, 28, and 30) in which the liquids A and B are a low concentration of an acid or a base, a sufficient yield for evaluation is achieved.

In the condition (No. 12) in which the liquid A is the PA peptide solution, the elution efficiency is low, and the evaluation concentration is significantly reduced, which makes it difficult to use for the evaluation of the physiological activity.

In the condition (No. 26) in which the size exclusion column purification step has been performed to remove the contaminant components after the elution, the peptide loss in the step of removing the contaminant components is large, and the evaluation concentration is significantly reduced, which made it difficult to use for evaluating the physiological activity.

The physiological activity of the polypeptide subjected to affinity purification and neutralization treatment under each condition was evaluated, in the same manner as in Example 1, using BaF3 cells stably expressing fibroblast growth factor receptor (FGFR). The present cells have properties of proliferating in the presence of various fibroblast growth factors (FGF) and analogous active molecules and dying in the absence thereof, and thus exhibits a proliferative activity in the presence of a polypeptide having bFGF-like physiological activity.

To allow the cell-free expression solution to act on cells at a lower dilution ratio, an RPMI medium (hereinafter, referred to as a "concentrated medium") 3 times more concentrated than the usual composition was prepared by mixing 750 µL of RPMI-1640 (10×) (Sigma-Aldrich, R1145), 750 µL of Fetal Bovine Serum (Thermo Fisher Scientific, 10270-106), 75 µL of Penicillin Streptomycin (Thermo Fisher Scientific, 15140-122), 75 µL of GlutaMAX (Thermo Fisher Scientific, 35050-061), 200 µL of Sodium Bicarbonate 7.5% solution (Thermo Fisher Scientific, 25080094), 75 µL of folic acid (FUJIFILM Wako Pure Chemical Corporation, 060-01802, dissolved to a concentration of 100 µg/mL in a 1% DMSO aqueous solution), 10 µL of Heparin Solution (2 mg/mL) (STEM CELL, 07980), and 565 µL of water. To evaluate the bFGF-like physiological activity of the polypeptide, the present concentrated culture medium does not contain bFGF, unlike the concentrated culture medium of Example 1.

The BaF3 cells stably expressing FGFR were washed twice with PBS, and then prepared to 4 × 10⁵ cells/mL using a culture medium obtained by mixing RPMI-1640 (containing L-glutamine and phenol red) (FUJIFILM Wako Pure Chemical Corporation, 189-02025) and Fetal Bovine Serum (Thermo Fisher Scientific, 10270-106) at a ratio of 10:1.

A liquid obtained by mixing 24 µL of the polypeptide which had been subjected to affinity purification and neutralization treatment under each condition with 12 µL of a concentrated culture medium was dispensed in 15 µL portions into a 384-well plate (Perkin Elmer, 6057300), 5 µL of the above-described cell suspension was then added to each well, and the mixture was shaken and mixed, and then cultured at 37°C (5% CO₂) for 2 nights. In this case, as a negative control having no physiological activity, wells were prepared in which 15 µL of a liquid obtained by mixing 24 µL of water with 12 µL of the concentrated culture medium was dispensed instead of the polypeptide.

After culturing for 2 nights, Hoechst 33342, Trihydrochloride, Trihydrate - 10 mg/mL Solution in Water (Thermo Fisher Scientific, H3570), -Cellstain (registered trademark) - Calcein-AM solution (1 mg/mL DMSO solution) (DOJINDO, C396), and -Cellstain (registered trademark) - PI solution (DOJINDO, P378) were each added to the RPMI (10% FBS) medium at a final concentration of 1/1000 by volume, and Live/dead staining was performed by allowing the mixture to stand at room temperature for 1 hour, and imaging was performed using a confocal microscope (Yokogawa Electric Corporation, CQ1). The number of viable cells was calculated from the imaging data and is shown in Table 4.

**[Table 3]**

| Results of evaluation of physiological activity of cell-free expressed polypeptides subjected to affinity purification and neutralization treatment | | | | | | | |
|---|---|---|---|---|---|---|---|
| No. | Cell-free expression solution | Purification | Affinity tag | Washing step | Liquid A | Liquid B | Number of viable cells |
| 2 | PUREfrex | Yes | PA | 2 times with PBS-T → 1 time with water | 10 mM hydrochloric acid | 100 mM sodium bicarbonate | 1,840 |
| 3 | PUREfrex | Yes | PA | 2 times with PBS-T → 1 time with water | 20 mM hydrochloric acid | 200 mM sodium bicarbonate | 2,630 |
| 4 | PUREfrex | Yes | PA | 2 times with PBS-T → 1 time with water | 30 mM hydrochloric acid | 300 mM sodium bicarbonate | 1,680 |
| 5 | PUREfrex | Yes | PA | 2 times with PBS-T → 1 time with water | 40 mM hydrochloric acid | 400 mM sodium bicarbonate | 2,302 |
| 11 | PUREfrex | Yes | PA | 3 times with PBS-T | 10 mM hvdrochloric acid | 100 mM sodium bicarbonate | 1,580 |
| 13 | PUREfrex | Yes | FLAG | 2 times with PBS-T → 1 time with water | 10 mM hydrochloric acid | 100 mM sodium bicarbonate | 1,640 |
| 14 | PUREfrex | Yes | HA | 2 times with PBS-T → 1 time with water | 10 mM hydrochloric acid | 100 mM sodium bicarbonate | 880 |
| 15 | PUREfrex | Yes | His | 2 times with PBS-T → 1 time with water | 10 mM hydrochloric acid | 100 mM sodium bicarbonate | 1,340 |
| 22 | PUREfrex | Yes | Strep | 2 times with PBS-T → 1 time with water | 10 mM hydrochloric acid | 100 mM sodium bicarbonate | 2,070 |
| 23 | PUREfrex | Yes | c-Myc | 2 times with PBS-T → 1 time with water | 10 mM hydrochloric acid | 100 mM sodium bicarbonate | 430 |
| 24 | PUREfrex | Yes | HA | 2 times with PBS-T → 1 time with water | 10 mM sodium hydroxide | 90 mM hydrochloric acid | 270 |
| 25 | PUREfrex | Yes | c-Myc | 2 times with PBS-T → 1 time with water | 10 mM sodium hydroxide | 90 mM hydrochloric acid | 140 |
| 28 | PURExpress | Yes | PA | 2 times with PBS-T → 1 time with water | 10 mM hydrochloric acid | 100 mM sodium bicarbonate | 25 |
| 30 | S30 | Yes | PA | 2 times with PBS-T → 1 time with water | 10 mM hydrochloric acid | 100 mM sodium bicarbonate | 45 |
| Negative control | | | | | | | 2 |

In each example, the viable cells were increased by 10 times or more as compared with the negative control, which indicates that the physiological activity of the polypeptide produced by a cell-free expression by the method according to the present invention can be evaluated.

The disclosure of JP2023-064440 filed on April 11, 2023 is incorporated in the present specification by reference in its entirety. All documents, patent applications, and technical standards described in the present specification are incorporated herein by reference to the same extent as in a case of being specifically and individually noted that individual documents, patent applications, and technical standards are incorporated herein by reference.
[Sequence list] International application 23F00024W1JP24014746_6.xml based on International Patent Cooperation Treaty

## Claims

1. A method for preparing a polypeptide solution for evaluating a physiological activity, the method comprising:
(A) bringing a crude polypeptide solution after cell-free expression into contact with a carrier to bind a polypeptide having a pH-responsive affinity tag to the carrier;
(B) eluting the polypeptide bound to the carrier with an acidic aqueous solution or a basic aqueous solution, each having a concentration of less than 50 mM, to obtain an eluted solution; and
(C) adding a basic aqueous solution or an acidic aqueous solution to the eluted solution to neutralize an acid or a base contained in the eluted solution, and neutralizing in such a manner that a concentration of a salt formed by a neutralization reaction is less than 50 mM, to obtain a polypeptide solution.

2. A method for evaluating a physiological activity of a polypeptide, the method comprising:
preparing a polypeptide solution by the method according to claim 1; and
bringing the polypeptide solution into contact with a cell to evaluate the physiological activity of the polypeptide.

3. The method according to claim 1 or 2,
wherein the polypeptide contains an unnatural amino acid residue.

4. The method according to claim 1 or 2,
wherein the polypeptide contains one or more ring structures.

5. The method according to claim 4,
wherein the ring structure includes the following structure, 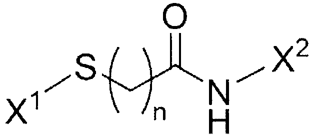
in the formula, X¹ and X² each independently represent any linking group, and n represents an integer of 1 to 10.

6. The method according to claim 4,
wherein the polypeptide contains 1 to 4 ring structures.

7. The method according to claim 1 or 2,
wherein, in (B), an acidic aqueous solution having a concentration of less than 50 mM is used, and, in (C), a basic aqueous solution is used.

8. The method according to claim 7,
wherein the acidic aqueous solution is an aqueous solution of hydrochloric acid.

9. The method according to claim 7,
wherein the basic aqueous solution is an aqueous solution of sodium bicarbonate, an aqueous solution of sodium carbonate, or a mixed aqueous solution of sodium bicarbonate and sodium carbonate.

10. The method according to claim 1 or 2,
wherein the carrier is magnetic beads.

11. The method according to claim 1 or 2,
wherein the affinity tag is a FLAG tag, an HA tag, a His tag, a c-Myc tag, a V5 tag, a Strep tag, or a PA tag.

12. The method according to claim 1 or 2, further comprising:
expressing the polypeptide by cell-free expression before (A).

13. The method according to claim 1 or 2, further comprising:
washing the carrier with water after (A) and before (B).
